(19) Europäisches Patentamt
European Patent Office
Office européen des brevets

(11) Publication number: 0 255 178 A2

# (12) EUROPEAN PATENT APPLICATION

(21) Application number: 87201430.3

(22) Date of filing: 24.07.87

(51) Int. Cl.4: C07C 51/377 , C07C 101/453 , C07D 209/34

(30) Priority: 01.08.86 IT 2138386

(43) Date of publication of application: 03.02.88 Bulletin 88/05

(84) Designated Contracting States: AT BE CH DE ES FR GB GR IT LI LU NL SE

(71) Applicant: BLASCHIM S.p.A.
Via Vittor Pisani, 28
I-20124 Milano(IT)

(72) Inventor: Citterio, Attilio
Piazzale Piola, 5
I-20131 Milano(IT)
Inventor: Fancelli, Daniele
Via Gianella, 21
I-20152 Milano(IT)
Inventor: Magni, Ambrogio
Via Donizetti, 20
I-22058 Osnago(IT)

(74) Representative: Marchi, Massimo et al
c/o Marchi & Mittler s.r.l. Viale Lombardia 20
I-20131 Milano(IT)

## (54) Process for preparing hetero aryl and aryl alkanoic acids.

(57) An aqueous solution comprising an alkali hydroxide, an aryl or a hetero aryl amine, substituted in ortho position by an alkanoic group and in para position by an inductive electron withdrawing atom or group, and an alkali nitrite is reacted with a mineral acid and hypophosphorous acid in an aqueous medium to afford an aryl and a heteroaryl alkanoic acid substituted in meta position by an electron withdrawing atom or group.

EP 0 255 178 A2

The present invention relates to a process for preparing hetero aryl and aryl alkanoic acids. More particularly, this invention relates to a proces comprising replacing an amino group in ortho position to an alkanoic group and in para position to an inductive electron withdrawing atom or group, with a hydrogen atom.

The term "inductive electron withdrawing" is used herein to indicate any substituent containing at least a hetero atom which withdraws electrons from the alkanoic group by an inductive effect; examples of such substituents are halogens, optionally substituted carbonyl groups, nitriles, ethers, thioethers, sulfides, sulfoxides, the sulfonic group, the nitro group and the trifluoromethyl group.

It is known that many aryl alkanoic acids, more particularly some aryl and hetero aryl acetic acids as well as some alpha aryl and alpha hetero aryl propionic acids, are endowed with valuable pharmacological properties and that their most common property is the anti-inflammatory one.

Since many of these drugs are used in the everyday practice, during the last twenty years investigations on new processes for producing them in a cheaper and cheaper way have become more frequent.

European patents 34,871 and 35,305 describe a process comprising substantially an acylation according to Friedel Crafts of a properly substituted aryl or hetero aryl compound to give the corresponding alpha halo alkyl ketone, preparation of the corresponding ketal, rearrangement of the latter and, finally, hydrolysis of the so obtained ester to give the desired aryl or hetero aryl alkanoic acid.

This method represents a break-through in the chemistry of aryl and hetero aryl alkanoic acids.

Nevertheless, it is known that the preparation of the intermediate ketones by the Friedel Crafts reaction shows serious drawbacks when the aryl and the hetero aryl moieties are substituted in meta position by an electron withdrawing group.

Typical examples of aryl alkanoic acids that are substituted in meta position by an electron withdrawing group are ketoprofen and fenoprofen of formula

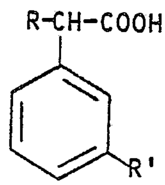

(I)

where R is methyl, and R' is benzoyl in the case of ketoprofen and phenoxy in the case of fenoprofen.

The inherent difficulties in finding a simple and cheap method for producing compounds such as fenoprofen and ketoprofen are also proved by the several attempts made up to now.

Most of the methods investigated so far comprise the preparation of a phenoxy benzene and, respectively, a benzoyl benzene substituted in meta position by an atom or a group, such as halogen, halo methylene, ethyl formyl, acetyl, methylene nitrile and the like, suitable to undergo conversion into the propionic group.

These methods involve many steps, give low yields and/or imply the use of expensive and/or polluting products and reactants.

Preparation procedures starting from an aryl or a hetero aryl compound substituted by a group that (i) promotes introduction of the alkanoic group on the aryl or hetero aryl ring, (ii) is able to direct introduction of an electrofile group in meta to the alkanoic group, and (iii) can easily be replaced by a hydrogen atom, have never been described.

The reason for this is probably that the directing group, which is more consistent with this synthesys pathway, is the amino group; in fact, this group allows the introduction of an alkanoic radical on the aryl moiety, in a rather simple way, according to the Stolle reaction and the so obtained aryl indolinone directs electrofile substitutions in meta to the alkanoic group. The subsequent treatment with alkalies leads then to compounds that, when the aryl moiety is benzene, have the following formula

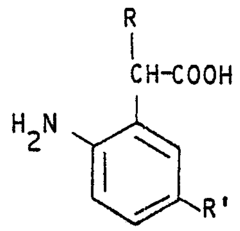

(II)

where R is H or alkyl, and

R' is an electron withdrawing group or atom.

Subsequent replacement of the amino group by a hydrogen atom, however, has never been described in spite of the fact that some compounds of formula II have been known for years.

It is known, on the other hand, that in order to obtain a deamined aromatic or hetero aromatic compound in good yields, the amine compound must be dissolved in an aqueous solution of a mineral acid and then treated with sodium nitrite; an aqueous solution of hypophosphorous acid is then added.

This method is not, however, useful in the case of amines of formula II because in acid medium cyclization into indolinone occurs according to the following scheme:

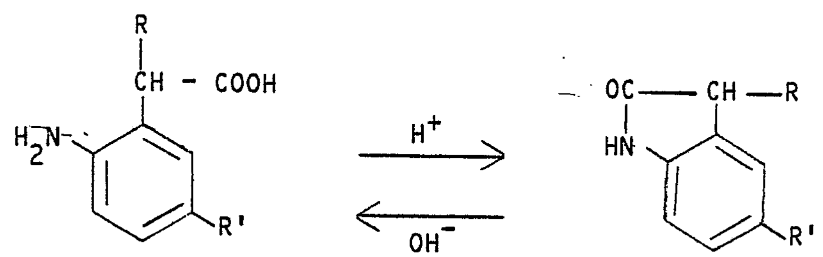


(II) (III)

where R and R' have the above mentioned meanings,

and the so obtained compound (III) does not undergo deamination.

It is also known that the yields are low and variable when, alternatively, an amine and sodium nitrite are dissolved or suspended in a solution of an alkali hydroxide and this solution or suspension is then poured into hydrochloric acid adding, finally, hyposphosphorous acid (R.H. Henry and W.G. Finnegan; J.A.C.S. 76,291, 1954). The Authors themselves report to have repeated the experiment several times obtaining yields varying from 26 to 46%.

On the other hand, the low yields are not correlated to a possible low reactivity of the tested amine since the Authors obtain a much higher yield (77.6%) when they add sodium nitrite to a suspension of the same amine in an aqueous solution of hypophosphorous acid.

According to the findings of these Authors the low yields from the first method are mostly due to the fact that, in those conditions, the amino group is partially replaced by a chlorine atom instead of by a hydrogen atom.

It has now been surprisingly found that primary aryl and hetero aryl amines substituted in ortho position by an alkanoic group and in para position by an inductive electron withdrawing atom or group, afford high yields of aryl and hetero aryl alkanoic acids when deamination is carried out in an alkaline medium.

It is, therefore, an object of the present invention to provide a process for preparing an aryl and a hetero aryl alkanoic acid substituted in meta position by an electron withdrawing group or atom, characterized in that an aqueous solution comprising an alkali hydroxide, an aryl or a hetero aryl amine, substituted in ortho position by an alkanoic group and in para position by an inductive electron withdrawing group or atom, and an alkali nitrite is reacted with a mineral acid and hypophosphorous acid in an aqueous medium.

The reaction of the alkaline solution with the mineral acid solution is preferably carried out at a temperature of from -15°C to -5°C.

The alkaline solution is preferably added slowly to the solution of the mineral acid and then hypophosphorous acid is added.

Optionally, hypophosphorous acid is added to the solution of the mineral acid before adding the alkaline solution.

To each mole of aryl or hetero aryl amine, from 1.05 to 1.2 moles of alkali nitrite and from 2 to 5 moles of hypophosphorous acid are preferably added.

To the aqueous solution of the mineral acid an organic solvent wherein the starting product and/or the product formed are soluble, may be added.

Examples of solvents of the first type are lower aliphatic alcohols such as methanol and isopropanol, cyclic ethers such as tetrahydrofuran and dioxane, and lower amides such as dimethyl formamide; examples of solvents of the second type are, on the contrary, chlorinated aliphatic hydrocarbons, such as methylene chloride, chloroform and 1,2-dichloro ethane, aromatic hydrocarbons such as toluene and xylene, cyclic aliphatic hydrocarbons such as cyclohexane, and their mixtures.

After the addition of hypophosphorous acid the reaction time may be reduced by adding a suitable catalyst, such as copper powder.

The amount of such a catalyst is preferably comprised from 1 to 3% by weight with respect to the amine that is reacted.

The reaction is then completed by allowing the temperature to rise to room temperature.

Some useful intermediates for preparing fenoprofen are new, and are, therefore, a further object of the present invention.

More precisely, the compounds of formula II and III where R is methyl and R' is phenoxy, are new.

It is, therefore, a further object of this invention to provide a compound of formula:

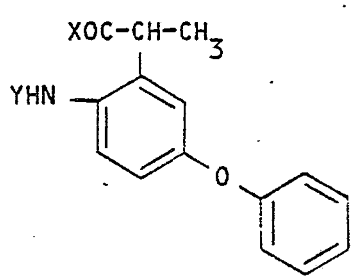

(IV)

wherein X is OH or, together with Y, is a covalent bond; and

Y is H when X is OH or, together with X, is a covalent bond;

and the salts with a base of the compound wherein X is OH and Y is hydrogen.

Typical examples of suitable bases are the alkali metals..

The following examples are intended to illustrate the present invention without, however, limiting it in any way.

EXAMPLE 1

2.14 g (7.97 moles) of 2-amino-5-benzoyl-alpha-methyl-phenylacetic acid (Belgian patent No. 837,154, Example 2, page 23) were dissolved in 30 ml of a 3N NaOH aqueous solution cooled to 0-5°C. To the so obtained solution, 0.61 g (8.8 mmoles) of sodium nitrite in 10 ml of water were added.

This solution was added, in about 55 minutes, to a solution of 20 ml of 18% hydrochloric acid kept under stirring at -10°C.

When the addition was over, 2.63 ml of 50% hypophosphorous acid (24 mmoles) were added while keeping the reaction mixture under stirring at -10/-5°C. The mixture was stirred allowing the temperature to rise to room temperature.

When the nitrogen bubbling was over, the reaction mixture was treated as described in Examples 4 below.

1.21 g of ketoprofen were so obtained; yield, 60%.

EXAMPLE 2

2.36 g (8.81 mmoles) of 2-amino-5-benzoyl-alpha-methyl-phenylacetic acid were reacted as described by R.A. Henry and W.Q. Finnegan (J.A.C.S. 76, page 291, left column, line 49 to 63).

The residue was then extracted as indicated in Example 4 below.

Yield, 20% (theor.).

EXAMPLE 3

2.00 g (7.46 mmoles) of 2-amino-5-benzoyl-alpha-methyl-phenylacetic acid were reacted as described in Example 1, except that variable amounts of copper powder (from 1 to 3%, with respect to the starting amine) were added after completion of the addition of hypophosphorous acid.

Yield, 62-64% (theor.).

EXAMPLE 4

8.57 g (0.032 mmoles) of 2-amino-5-benzoyl-alpha-methyl-phenylacetic acid (Belgian patent No. 837,154, Example 2, page 23) were dissolved in 120 ml of an aqueous solution of 3N NaOH and cooled to 5-15°C. To the so obtained solution, 2,31 g (0.033 moles) of sodium nitrite in 30 ml of water were added.

This solution was added, in about 50 minutes, to a solution of 38 ml of 35% hydrochloric acid and 120 ml of methanol kept under stirring at -10/-5°C; when the addition was over, 10.5 ml of hypophosphorous acid were added while keeping the reaction mixture under stirring at a temperature of from -10 to -5°C, and then 62 mg of copper powder were added. The mixture was maintained under stirring allowing the temperature to rise to room temperature.

When the nitrogen bubbling was over, the reaction mixture was extracted with ethyl ether (3×60 ml).

The organic extracts were washed with water (50 ml), dried on sodium sulfate and evaporated.

After standing overnight in a refrigerator, the oily residue gave a crystalline product (ketoprofen) which was then purified by recrystallization from isopropyl ether/pentane (1:1).

Yield, 6.64 g (82%); m.p. 93-94°C.

In a similar way, the following compounds were prepared:

-5-benzoyl-phenylacetic acid (starting compound: 2-amino-5 benzoyl-phenylacetic acid, Belgian Patent 837,154, Example 1, page 23). Yield, 79%; m.p. 113-114°C (toluene/pentane);

-3-bromo-alpha-methylphenylacetic acid, yield: 81%; m.p. 69-70°C from (hexane); starting compound: 2-amino-5-bromo-alpha-methylphenylacetic acid (prepared similarly to 2-amino-5-benzoylphenylacetic acid; m.p. 101-102°C); 3-bromo-alpha-methylphenylacetic acid was then transformed into fenoprofen by reaction with potassium phenate.

EXAMPLE 5

2.1 g (7.84 mmoles) of 2-amino-5-benzoyl-alpha-methylphenylacetic were dissolved in 3N sodium hydroxide (40 ml) to which a solution of 0.58 g (7.7 mmoles) of sodium nitrite has been added in advance.

This solution was cooled to 5°C and then added under stirring in 90 minutes to a mixture of 10 ml of 35% hydrochloric acid, 10 ml of water, 2.6 g (24 mmoles) of hypophosphorous acid, 25 mg of copper powder and 10 ml of toluene, cooled to -10/-5°C.

The stirring was continued allowing the temperature to rise to room temperature.

The organic phase was separated and the aqueous phase was extracted with toluene (10 ml). The combined toluene extracts were extracted with a 1N sodium hydroxide solution (15ml).

The aqueous phase was separated and acidified at 0°C.

The precipitate was collected, dried at 40°C under vacuum for 5 hours and then recrystallized from benzene/cyclohexane (1:6).

1.30 g of ketoprofen were so obtained. Yield, 71%.

EXAMPLE 6

4-phenoxy-alpha-chloropropionanilide

Alpha-chloro propionyl chloride (13.4 g; 0.11 moles) was added to a solution of freshly recrystallized 4-phenoxyaniline (m.p. 83-84°C; 18.5 g; 0.1 moles) and triethyl amine (10.7 g; 0.11 moles) in ethyl ether (200 ml), under stirring at 10°C. After 1 hour, water was added (100 ml); the organic phase was separated and the aqueous phase extracted with ethyl ether (50 ml).

The etheral phase was washed with a saturate sodium chloride solution (50 ml), dried and evaporated at 30°C to give a solid which was recrystallized from methanol (1:1); m.p. 107°C.

Yield, 24.3 g, (88%).

I.R. ($\nu$,$cm^{-1}$): 3280 (NH), 1660 (CONH)

5-phenoxy-3-methylindolin-3-one

13.8 g (0.05 moles) of 4-phenoxy-alpha-chloroanilide were added to 53.3 g of $AlCl_3$ at 60°C under vigorous stirring. The melted mass was heated to 150°C for 45 minutes under nitrogen, and then added to a mixture of water and chloroform (100:50 ml) under stirring.

The organic phase was separated and extracted with chloroform (2×2 ml). The chloroform extracts were washed with water, dried on sodium sulfate and evaporated.

The residue was chromatographed on "flash" silica eluting with methylene chloride/ethyl acetate (8:2). 3.6 g (30%) of 5-phenoxy-3-methylindolin-2-one were so obtained;

m.p. 150-151°C (methyl alcohol).

El. anal.: Teor.: C, 75.30; H, 5.48; N, 5.85

Found: C, 75.4; H, 5.6; N, 5.9

H-NMR ($CDCl_3$, delta): 9.1 (wide, 1H, NH); 6.9-7.4 (m, 8H, Ar), 3.45 (quartet, 1H, $CHCH_3$); 1,55 (d, 3H, $CHCH_3$).

I.R. (nujol, $\nu$ $cm^{-1}$): 3160 (wide, NH), 1710 ( CO)

M.S. (m/e); 239 ($M^+$, 100), 234(6), 211(47), 146(15), 134(32).

Fenoprofen

5-phenoxy-3-methylindolin-2-one (4.78 g, 0.02 moles) was suspended in a 4N sodium hydroxide solution (100 ml) and the mixture was refluxed for 24 hours under nitrogen. The reaction mixture was cooled and extracted with chloroform at 10°C (2×50 ml). The aqueous phase was cooled to -5/-10°C and 10% hydrochloric acid was added to pH 6. The mixture was extracted with cold chloroform (3×40 ml) and the extracts were dried and evaporated. The residue (2-amino-5-phenoxy-alpha-methyl-phenylacetic acid) was crystallized from ethanol/water.

The so obtained product was deamined in a way similar to that described in Example 4, except that the final product was distilled under vacuum (0.05 mm Hg).

Yield, 72%; b.p. 160-162°C

**Claims**

1. A process for preparing an aryl and a hetero aryl alcanoic acid substituted in meta position by an electron withdrawing group or atom, characterized in that an aqueous solution comprising an alkali hydroxide, an aryl or a hetero aryl amine substituted in ortho position by an alkanoic group and in para position by an inductive electron withdrawing group or atom, and an alkali nitrite is reacted with a mineral acid and hypophosphorous acid in an aqueous medium.

2. A process according to claim 1, characterized in that the reaction of the alkaline solution with the solution of the mineral acid is carried out at a temperature of from -15 to -5°C.

3. A process according to any of the claims 1 and 2, characterized in that the reaction of the alkaline solution with the solution of the mineral acid is carried out by adding slowly the alkali solution to the solution containing the mineral acid.

4. A process according to any of the claims from 1 to 3, characterized in that the alkaline solution is first reacted with the solution of the mineral acid and then with hypophosphorous acid.

5. A process according to any of the claims from 1 to 3, characterized in that the alkaline solution is reacted with a solution containing both the mineral acid and the hypophosphorous acid.

6. A process according to any of the claims from 1 to 5, characterized in that each mole of an aryl or a hetero aryl amine is reacted with from 1 to 1.2 moles of alkali nitrite and from 2 to 5 moles of hypophosphorous acid.

7. A process according to any of the claims from 1 to 6, characterized in that the aqueous solution of the mineral acid comprises also an organic solvent.

8. A process according to claim 7, characterized in that the organic solvent is selected from lower aliphatic alcohols, cyclic ethers, lower amides, chlorinated aliphatic hydrocarbons, aromatic hydrocarbons, cyclic aliphatic hydrocarbons, and mixture thereof.

9. A process according to any of the claims from 1 to 8, characterized in that after hypophosphorous acid, copper powder is added.

10. A process according to claim 9, characterized in that the amount of copper powder ranges from 1 to 3% by weight, with respect to the amine compound.

11. A process according to any of the claims from 1 to 10, characterized in that in the aryl and hetero aryl amine, the alkanoic group in ortho position to the amino group is $-CH_2-COOH$ or $-CH(CH_3)-COOH$, and the inductive electron withdrawing group or atom in para position to the amino group is halogen, an optionally substituted carbonyl, a nitrile, an ether, a thioether, a sulfone, a sulfoxide, a sulfonic group, a nitro group or a trifluoromethyl group.

12. A process according to claim 11, characterized in that in the aryl and hetero aryl amine, the inductive electron withdrawing group or atom in para position to the amino group is bromine, phenoxy or benzoyl.

13. A compound of formula:

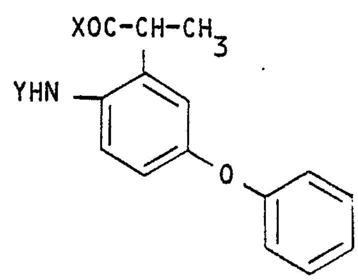

(IV)

where X is OH or, together with Y, forms a covalent bond;

Y is H when X is OH or, together with Y, has the above mentioned meaning;
and salts with a base of the compound where Y is H and X is OH.